# EUROPÄISCHE PATENTSCHRIFT

(11) **EP 2 745 785 B1**
(45) Veröffentlichungstag und Bekanntmachung des Hinweises auf die Patenterteilung: **21.06.2017**
(21) Anmeldenummer: 12405128.5
(22) Anmeldetag: 21.12.2012
(51) Int. Cl.: A61B 17/12, A61F 13/02, A61B 17/132, A61B 17/00, A61F 13/00, A61B 90/00

(54) **Gefässverschlusssystem**
Vessel closure system
Système de fermeture de récipient

(43) Veröffentlichungstag der Anmeldung: 25.06.2014
(73) Patentinhaber: VOSTRA-MED AG, 6330 Cham (CH)
(72) Erfinder: Harren, Ernst-Diethelm, 8570 Weinfelden (CH); Ackermann, Simon, 8442 Hettlingen (CH)
(74) Vertreter: Luchs, Willi

(56) Entgegenhaltungen:
- DE-U1-202011 106 809
- US-A- 4 592 342
- US-A- 5 312 350
- US-B1- 6 274 786

## Beschreibung

Die Erfindung betrifft ein Gefässverschlusssystem nach Patentanspruch 1. Insbesondere betrifft die Erfindung ein Gefässverschlusssystem zur Sicherung der Punktionsstelle in der Haut und der Einstichstelle eröffneter Gefässe vor Nachblutungen, und welches der permanenten visuellen Überprüfung des ungehinderten Blutflusses im Gefäss dient.

Die Erfindung betrifft insbesondere ein ortsdefiniert aufklebbares Gefässverschlusssystem zur Sicherung eröffneter Gefässe vor Nachblutungen mit mindestens einem Druckkörper, der geeignet ist, mit einer Wunde in Berührung zu gelangen. Gefässverschlusssysteme sind wirksam in Bezug auf die Zeit bis zur Blutstillung. In diesem Bereich der medizinischen Anwendungen, insbesondere aber bei der Eröffnung von Gefässen für interventionelle Eingriffe, gibt es noch immer einen Bedarf, eine auch für medizinisches Fachpersonal einfach zu handhabende Sicherung eröffneter Gefässe zur Verfügung zu stellen, mit der nicht nur ein späterer Blutaustritt bzw. Blutverlust in den freien Raum ausserhalb des Körpers, sondern auch der Blutaustritt in das Gewebe (Hämatombildung) verhindert wird, wobei gleichzeitig aber auch die lebenswichtige Blutzirkulation innerhalb des Körpers nach erfolgtem Einsatz und endgültigem Anlegen des Gefässverschlusssystems nachweisbar nicht behindert werden soll.

In besonderer Weise geschieht das Verschliessen von eröffneten Gefässen nach interventionellen Eingriffen, indem ein Verschlusssystem implantiert wird. Diese sind jedoch sehr teuer und haben zur Folge, dass mindestens über eine Zeitdauer von einem halben Jahr an dieser Stelle kein Zugang für interventionelle Eingriffe geschaffen werden kann.

In herkömmlicher Weise geschieht das Verschliessen eröffneter Gefässe derart, dass nach dem Eingriff an der Punktionsstelle und der Gefässeinstichstelle gleichzeitig im richtigen Mass manueller Druck ausgeübt wird. Wegen der Gefahr der Hämatombildung darf der Druck nicht zu klein sein, wegen der Gefahr der Kreislaufunterbindung im betroffenen Körperteil aber auch nicht zu gross.

Nachdem eine Blutstillung erreicht ist, werden mechanische Hilfsmittel derart mit Verbänden am Körper befestigt, dass das mechanische Hilfsmittel in Verbindung mit dem Verband die weitere Kompression übernimmt.

Anschliessend werden die Patienten in einen Spitaltrakt mit intensiver Überwachung zur Beobachtung verbracht. Die Beobachtung ist jedoch auf die Beschau von Verbandstoffen eingeschränkt. Sollte nun der Verband rot gefärbt sein, so kann die behandelnde medizinische Fachkraft daraus schliessen, dass der Patient einen starken Blutverlust erleidet. Der Verband muss dann entfernt werden. Sollte die Einstichstelle wegen heftigem Blutaustritt nicht sofort detektiert werden können, so kann das Blut bis zum Auffinden der Punktionsstelle in den freien Raum ausströmen. Nachdem die Punktionsstelle gefunden ist, beginnt das Abdrücken und Versorgen wieder wie zuvor beschrieben.

In der Regel werden die Patienten nach ca. 6 Stunden (Aufenthalt im Spitaltrakt mit intensiver Überwachung) ohne komprimierende Verbände entlassen.

Nachteilig bei Gefässverschlusssystemen aus dem Stand der Technik sind oftmals auftretende Komplikationen, wie z.B. Hämatome und Pseudoaneurysma-Bildung. Nachteilig ist zudem eine lange Verweildauer der Patienten unter Beobachtung im Krankenhaus, wodurch hohe Kosten entstehen.

Eine Verschlussvorrichtung gemäss der Druckschrift DE-Gbm-20 2011 106 809 zum Verschliessen eines eröffneten Blutgefässes weist die Merkmale des Oberbegriffs von Anspruch 1 auf und ist mit einem Verschlusselement und einem Druckkörper versehen, wobei das Verschlusselement aus einem Material mit elastischen Eigenschaften ausgebildet ist. Es ist eine Vielzahl von Haltebändern vorgesehen, welche an distalen Enden des Verschlusselementes anschliessen, wobei die Haltebänder unelastische Eigenschaften haben. Mit der halbkugelförmigen Ausbildung des Druckkörpers dieser sich bewährten Verschlussvorrichtung bestehen gewisse Nachteile in Bezug auf die punktuelle Druckstelle, welche bei einem Gefässverschliessen entsteht.

Es ist Aufgabe der vorliegenden Erfindung ein komplikationsfreies Gefässverschlusssystem aufzuzeigen, welches eine kurze Verweildauer des Patienten im Krankenhaus gestattet.

Diese Aufgabe wird durch ein Gefässverschlusssystem nach den Merkmalen des Anspruchs 1 gelöst. Erfindungsgemäss ist der wenigstens eine Druckkörper ausgelegt, im angelegten Zustand an einem eröffneten Gefäss bei einem Patienten, einen in Relation zu weiteren Bereichen des Gefässes erhöhten Druck an eine Einstichstelle im Gefäss anzulegen.

Das erfindungsgemässe Gefässverschlusssystem gestattet es überraschend einfach, einen individuell durch Zugkraft auf das Gefässverschlusssystem einstellbaren Druck auf die Gefässöffnung anzulegen. Ferner ist auf einfache Weise ein Gefässverschlusssystem geschaffen, bei welchem eine unterschiedliche Verschlusselementgeometrie zur Verfügung gestellt ist. Im Gegensatz zu Verschlusssystemen aus dem Stand der Technik kann somit berücksichtigt werden, dass sich die Punktionsstelle auf der Haut und die Gefässeinstichstelle auf unterschiedlichen Ebenen befinden.

Nachdem das erfindungsgemässe Gefässverschlusssystem angelegt ist, werden die Patienten in einen Spitaltrakt mit intensiver Überwachung zur Beobachtung verbracht. Im Gegensatz zu Gefässverschlusssystemen aus dem Stand der Technik ist diese Beobachtungs-Verweildauer mit intensiver Überwachung verkürzt, wodurch Kosten eingespart werden.

Sollte es nun zu einer Nachblutung kommen, kann die medizinische Fachkraft durch ein Drücken auf der Rückseite des Druckkörpers eine temporäre, unterstützende Kompression vornehmen. Beim Verlassen der medizinischen Einrichtung können die Patienten das Gefässverschlusssystem auf der Einstichstelle belassen. Für den Fall, dass es ausserhalb der medizinischen Einrichtung zu einer Nachblutung kommt, kann der Patient auf gleiche Art und Weise wie zuvor beschrieben selbst eine temporäre, unterstützende Kompression anwenden. Das Entfernen des Gefässverschlusssystems übernimmt der Patient zum gegebenen Zeitpunkt. Bis dahin bleibt die Punktionsstelle sicher und hygienisch verschlossen. Hierdurch sind bislang ungeahnte Einsparungen im Gesundheitssektor realisierbar.

Vorzugsweise ist der wenigstens eine Druckkörper ausgelegt einen in Relation zur Einstichstelle im Gefäss reduzierten Druck an eine Punktionsstelle an der Haut anzulegen. Somit werden arterielle Blutstauungen im Gefäss verhindert, welche bei herkömmlichen Gefässverschlusssystemen auftreten.

Vorzugsweise ist der wenigstens eine Druckkörper ausgelegt einen in Relation zur Einstichstelle im Gefäss reduzierten Druck an einen Bereich zur Pulsmessung am Gefäss anzulegen. Durch dieses Merkmal kann das Gefässverschlusssystem mit einem Pulsmesssensor erweitert werden, ohne dass arterielle Blutstauungen auftreten werden. Der transparente Druckkörper lässt die Beobachtung der Punktionsstelle uneingeschränkt zu. Ebenfalls besteht durch Anschliessen eines Blutdruckmessgeräts die Möglichkeit, per Messfühler den ungehinderten Blutfluss im Gefäss permanent zu überwachen.

Vorzugsweise ist der wenigstens eine Druckkörper der anatomischen Form einer Fingerabbildung zum manuellen Verschliessen des eröffneten Gefässes nachgebildet. Beim Einsatz eines solchen Gefässverschlusssystems drückt der behandelnde Arzt nach dem invasiven Eingriff die Punktionsstelle in der Haut und die Gefässeinstichstelle solange gleichzeitig manuell ab, bis die Blutflussstillung erreicht ist. Hiernach platziert der behandelnde Arzt das Gefässverschlusssystem derart, dass sowohl die Punktionsstelle in der Haut als auch die Gefässeinstichstelle zur Abdeckung kommen.

Vorzugsweise entspricht die anatomische Form einer Nachbildung von drei Fingern. Weiter vorzugsweise steht die mittlere Fingernachbildung der drei Fingernachbildungen in Relation zu den beidseitig anliegenden Fingernachbildungen anatomisch nachgebildet vor. Hierdurch ist ein Gefässverschlusssystem geschaffen, bei welchem die Einstichstelle im Gefäss, die Punktionsstelle an der Haut und ein Bereich zur Pulsmessung zugleich auf optimale Weise abgedrückt werden. Vorzugsweise ist die anatomische Form der Fingernachbildungen in Bezug auf eine Einstichstelle im Gefäss, eine Punktionsstelle an der Haut und einen Bereich der Pulsmessung am Gefäss abgestimmt. Erfingdungsgemäß hat der wenigstens eine Druckkörper eine treppenförmige Aussenform. Hierdurch ist ein weicher Übergang von einem erhöhten (maximalen) Abdrücken an der Einstichstelle im Gefäss, zu einem verringerten Abdrücken an der Punktionsstelle an der Haut, zu einem weiter verringerten Abdrücken an weiteren Bereichen am Gefäss möglich. Durch diese Ausgestaltung können Bereiche am Gefäss mit einem unterschiedlichen Druck abgedrückt werden. Eine grösste Erhebung der treppenförmigen Aussenform drückt hierbei die Einstichstelle im Gefäss ab, während weitere Erhebung der treppenförmigen Aussenform dazu ausgelegt sind, weitere Bereiche am Gefäss abzudrücken.

In einer besonderen Ausführungsform sind wenigstens zwei der Druckkörper (3) im rechten Winkel zur Längserstreckung der Haltebänder (4) angeordnet. Erfindungsgemäß enthält das Gefässverschlusssystem ferner eine Vielzahl von Haltebändern, welche an distalen Enden des Verschlusselementes anschliessen, wobei die Haltebänder unelastische Eigenschaften haben, und vorzugsweise zumindest ein Indikatorelement, welches mit dem Verschlusselement verbunden ist. Das Gefässverschlusssystem gestattet es überraschend einfach, einen individuell durch Zugkraft einstellbaren Druck auf die Einstichstelle im Gefäss, die Punktionsstelle an der Haut und an einen Bereich zur Pulsmessung am Gefäss anzulegen. Die hierzu nötige Zugkraft lässt sich, noch bevor das Gefässverschlusssystem aufgeklebt wird, anhand des Indikatorelements ablesen. Zur besseren Ablesbarkeit sind zumindest zwei Indikatorelemente bereitgestellt. Mit anderen Worten, ist es möglich, anhand der veränderlichen Form bzw. Geometrie der Indikatorelemente, welche durch veränderliche Zugkraft auf das Gefässverschlusssystem hervorgerufen wird, einen Rückschluss auf den jeweiligen Druck auf die Einstichstelle im Gefäss zu ziehen, sobald das Gefässverschlusssystem in dem (unter Zugkraft gesetzten) Zustand aufgeklebt ist.

Eine hohe Präzision in der Ablesbarkeit der Indikatorelemente wird überraschend einfach und zuverlässig dadurch erreicht, indem das Verschlusselement elastische Eigenschaften hat und die Haltebänder wiederum unelastische Eigenschaften haben. Es ist hierbei zu erwähnen, dass als Haltebänder jeweils Ausläufer an den Enden des Verschlusselements definiert sind. An diesen Haltebändern ergreift der Benutzer das Gefässverschlusssystem, beispielsweise zwischen Zeigefinger und Daumen, und übt durch Strecken des Gefässverschlusssystems eine Zugkraft auf das Gefässverschlusssystem aus. Diese Zugkraft wird wiederrum fast ausschliesslich durch das Material des Verschlusselementes aufgenommen, welches elastische Eigenschaften besitzt.

Das Gefässverschlusssystem erlaubt eine einfache und sichere Handhabung. Ausserdem kann der an der Einstichstelle im Gefäss angelegte Druck durch Ablesen der Indikatorelemente individuell und anwendungsspezifisch eingestellt werden. Somit ist immer der korrekte Druck an der Einstichstelle im Gefäss, an der Punktionsstelle auf der Haut und an dem Bereich zur Pulsmessung am Gefäss einstellbar. Zudem ist der Aufbau des Gefässverschlusssystems sehr einfach, wodurch die Herstellungskosten gesenkt werden.

Vorzugsweise sind das Verschlusselement und die Haltebänder einstückig ausgebildet. Das Gefässverschlusssystem ist hierbei mit geeignet langen Haltebändern versehen. Neben der guten Handhabbarkeit soll damit auch eine genügende Haftung auf der Haut erreicht werden, um den zur Blutstillung nötigen Gegendruck aufbauen zu können. Die Geometrie des Gefässverschlusssystems ist nicht an eine solche Form gebunden. Andere Formen sind ebenso denkbar, wie beispielsweise kreisförmige Patches. Allerdings, wie zuvor erwähnt, muss das Verschlusselement eine elastische Eigenschaft aufweisen und müssen die Haltebänder eine unelastische Eigenschaft aufweisen. Hierdurch lässt sich die notwendige Zugkraft anhand der Indikatorelemente ablesen, um somit einen individuell notwendigen Druck an die Einstichstelle im Gefäss anlegen zu können.

Alternativ sind das Verschlusselement und die Haltebänder mehrstückig ausgebildet. In dieser Ausführungsform können das Verschlusselement aus einem elastischen Material und die Vielzahl von Haltebändern aus einem unelastischen Material durch geeignete Verbindungsverfahren miteinander verbunden werden.

Vorzugsweise sind das Verschlusselement und/oder der wenigstens eine Druckkörper und/oder das wenigstens eine Indikatorelement einstückig ausgebildet. Durch die Ausformung dieser Elemente aus einem einzigen Material sind hohe Kosteneinsparungen erzielbar. Zudem sind hohe Stückzahlen in kurzer Zeit herstellbar.

Vorzugsweise ist das zumindest eine Indikatorelement dazu ausgelegt mit zunehmender Streckung durch Anlegen einer Zugkraft auf das Verschlusselement eine veränderte Geometrie anzunehmen. Hierdurch kann in Ansprechen auf einen gewünschten Druck, welcher an die Einstichstelle im Gefäss anzulegen ist, eine derartige Zugkraft an die Enden des Gefässverschlusssystems angelegt werden, bis die Indikatorelemente eine hierzu notwendige (definierte) Geometrie annehmen.

Sobald der Benutzer die hierzu definierte Geometrie der Indikatorelemente optisch abliest bzw. erkennt, ist die korrekte Zugkraft angelegt und kann das mit dieser Zugkraft gestreckte Gefässverschlusssystem auf die Haut des Patienten angebracht werden. Hierdurch ist es vorteilhafterweise möglich, mit einfachen Mitteln einen individuellen Druck an der Einstichstelle im Gefäss und an der Punktionsstelle an der Haut anzulegen.

Vorzugsweise nimmt das zumindest eine Indikatorelement in einem unbelasteten Zustand des Verschlusselements jeweils die Geometrie einer ovalen Form an, welche mit zunehmender Streckung des Verschlusselements in eine zunehmend kreisförmige Form übergeht und mit weiter zunehmender Streckung in eine zunehmend ovale Form übergeht. Durch die Ausgestaltung der Indikatorelemente in ovaler Form ist eine besonders zuverlässige Einstellung der jeweils notwendigen Zugkraft auf das Gefässverschlusssystem durch Inaugenscheinnahme der sich veränderlichen Form möglich. In Abhängigkeit von der medizinischen Indikation stellt sich eine veränderte Geometrie ein. Durch Strecken des Gefässverschlusssystems wird die anfangs ovale Form der Indikatorelemente zunehmend rund. Durch ein Strecken des Gefässverschlusssystems über diesen gestreckten Zustand hinaus ändert sich die Geometrie der Indikatorelemente von der runden Form auf eine ovale Form. Durch Ablesen der veränderlichen Form kann die nötige Zugkraft einfach und zuverlässig definiert werden.

Vorzugsweise enthält das Gefässverschlusssystem ferner ein flüssigkeitsabsorbierendes Element, welches umfangsseitig um den wenigstens einen Druckkörper angeordnet ist. Dieses flüssigkeitsabsorbierende Element saugt möglicherweise aus der Nadel austretendes Blut auf. Das Gefässverschlusssystem hat auch unter hygienischen Aspekten Vorteile gegenüber bekannten und gängigen Lösungen. Das Gefässverschlusssystem ist als steriles Einwegprodukt vorgesehen, um insbesondere auch Kreuzinfektionen auszuschliessen.

Vorzugsweise enthält das flüssigkeitsabsorbierende Element einen Wirkstoff mit schmerzstillenden und/oder blutstillenden und/oder antiallergischen Wirkstoffen. Hierdurch wird eine gute Verträglichkeit auch bei langandauernder Applikation des Gefässverschlusssystems am Patienten gewährleistet.

Vorzugsweise ist jeweils in dem Bereich der Haltebänder, auf einer Seite des Gefässverschlusssystems, auf welcher der wenigstens eine Druckkörper bereitgestellt ist, eine Verstärkungsschicht angebracht, welche aus einem Material mit unelastischen Eigenschaften ausgebildet ist. Mittels dieser Verstärkungsschicht werden den Haltebändern die notwendigen unelastischen Eigenschaften vermittelt. Hierbei ist zu verstehen, dass unter einem Material mit unelastischen Eigenschaften ein Material zu verstehen ist, das keine Erstreckung in Längs- als auch in Querrichtung zulässt. Wie anhand der vorherigen Beschreibung des Anwendungsbereiches zu verstehen, ist selbstverständlich eine derartige Verformbarkeit des Materials gegeben, welche ein Anschmiegen und Verkleben der Haltebänder an auch nicht ebenen Körperbereichen des Menschen zulässt.

Vorzugsweise ist die Verstärkungsschicht mittels eines Klebemittels jeweils an einer Seite mit dem Bereich der Haltebänder verbunden und auf der weiteren Seite mit einer Hautkleberschicht bereitgestellt. Mit Hilfe der Hautkleberschicht wird das Gefässverschlusssystem mittels Klebekraft derart an der Haut des Patienten fixiert, dass der Druckkörper die Punktionsöffnung hierdurch zuverlässig verschliesst.

Vorzugsweise ist die Hautkleberschicht mit einer abziehbaren Schutzfolie bedeckt. Hierdurch wird die Hautkleberschicht geschützt und kann somit eine langandauernde Klebekraft der Hautkleberschicht gewährleistet werden. Dem Anlegen des Gefässverschlusssystems auf die Hautfläche des Patienten geht lediglich ein Abziehen der Schutzfolie von der Hautkleberschicht voraus.

Vorzugsweise ist die Schutzfolie verschiedenfarbig. Ferner vorzugsweise kennzeichnen die verschiedenen Farben den Einsatzbereich des Gefässverschlusssystems. Hierdurch ist eine eindeutige Indikation möglich.

Vorzugsweise bestehen das Verschlusselement und/oder der wenigstens eine Druckkörper und/oder die Haltebänder und/oder das wenigstens eine Indikatorelement aus Silikon, Naturkautschuk, Kunstkautschuk, Gummi, Latex, Hydrogel, polymerem Kunststoff oder einer Kombination dieser Materialien. Wie zuvor beschrieben, besitzen die Materialien des Verschlusselements eine hohe Elastizität und weisen eine gewünscht hohe Rückstellkraft auf. Insbesondere ist Silikon wegen der hervorragenden medizinischen Eignung ein bevorzugtes Material für das Gefässverschlusssystem. Ein Vorteil in der Auswahl von Silikon besteht ferner darin, dass die vorhandene ausserordentlich hohe Dehnfähigkeit in der flächigen Ausdehnbarkeit begrenzt und stabilisiert ist. Dies ist notwendig, um den zur Blutstillung notwendigen, individuell einstellbaren Gegendruck aufbringen zu können.

Vorzugsweise sind das Verschlusselement und/oder der wenigstens eine Druckkörper und/oder die Haltebänder und/oder das wenigstens eine Indikatorelement und/oder die Verstärkungsschicht transparent oder annähernd transparent. Hierdurch wird das Aufbringen des Gefässverschlusssystems als Punktionsverschluss an korrekter Position erleichtert. Ein transparenter Druckkörper lässt die Beobachtung der Punktionsstelle uneingeschränkt zu.

Vorzugsweise sind die Haltebänder wasserdampfdurchlässig. Hierdurch wird die Trageeigenschaft des Gefässverschlusssystems insgesamt verbessert.

Vorzugsweise sind die Haltebänder perforiert. Durch die Perforierungen kann Wasserdampf schnell abgeführt werden, was die Trageeigenschaft des Gefässverschlusssystems verbessert. Vorzugsweise sind die Haltebänder verlängerbar oder verkürzbar. Somit kann das Gefässverschlusssystem an unterschiedlichen Stellen am Körper des Patienten zuverlässig angebracht werden.

Vorzugsweise enthält das Gefässverschlusssystem ferner ein Indikatorelement zur Anzeige einer maximalen Verkürzung. Ferner vorzugsweise ist die Verkürzung der Haltebänder eingeschränkt. Somit ist das Gefässverschlusssystem mit stets ausreichender Zugspannung am Patienten angebracht.

Vorzugsweise enthält das Gefässverschlusssystem ferner wenigstens ein zusätzliches Verlängerungshalteband. Durch die Möglichkeit der Verlängerung ist das Gefässverschlusssystem auch bei adipösen Patienten anwendbar. Das Verlängerungshalteband wird mit dem Gefässverschlusssystem verbunden, bevor es appliziert wird. Das Verlängerungshalteband muss eine vordere Klebefläche zur Verbindung mit dem Gefässverschlusssystem und eine hintere Klebefläche zur Verbindung mit der Haut enthalten.

Vorzugsweise enthält das Gefässverschlusssystem ferner eine Aufnahme für einen Pulsfrequenzfühler.

Die Anfangs genannte Aufgabe wird ebenfalls durch eine Verwendung eines Gefässverschlusssystems nach einem der Ansprüche 1 bis 30 zum Verschliessen eines eröffneten Gefässes direkt nach einem Primärverschluss oder nach einem vorherigen händischem Abdrücken oder vor einem Verlassen einer klinischen- oder polyklinischen Einrichtung zur Sicherung eines eröffneten Gefässes gegen Infektionen oder Nachblutungen nach einem interventionellen Eingriff gelöst.

Im Folgenden wird das erfindungsgemässe Gefässverschlusssystem anhand der Zeichnung detailliert beschrieben.

In den Zeichnungen zeigen:
- Fig. 1: einen Längsschnitt durch ein ortsdefiniert aufklebbares Gefässverschlusssystem;
- Fig. 2: eine Ansicht auf das Gefässverschlusssystem gemäss Fig. 1;
- Fig. 3: eine schematische Skizze zur Anwendung des Gefässverschlusssystems; und
- Fig. 4a-c: schematische Ansichten des Gefässverschlusssystems bei unterschiedlichen Streckungen.

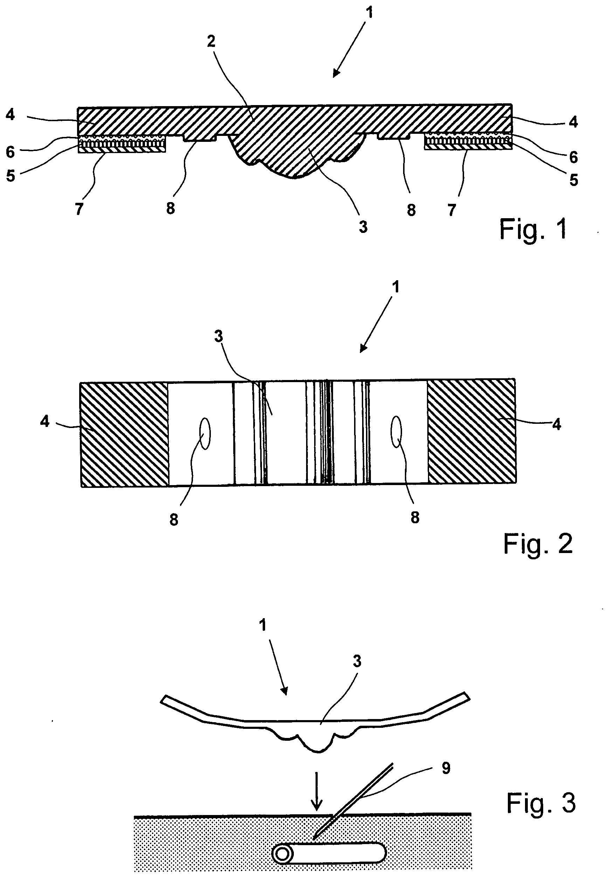

Die Fig. 1 zeigt beispielhaft einen nicht-massstäblichen Längsschnitt durch ein ortsdefiniert aufklebbares Gefässverschlusssystem 1, und Fig. 2 zeigt hierzu eine Ansicht von unten auf das Gefässverschlusssystem 1 gemäss Fig. 1. Das Gefässverschlusssystem 1 enthält ein Verschlusselement 2 und wenigstens einen Druckkörper 3, welche in diesem Beispiel einstückig aus einem Material mit elastischen Eigenschaften, beispielsweise Silikon, ausgebildet sind. Der Druckkörper 3 ist derart geformt, dass er dazu ausgelegt ist, im angelegten Zustand an einem eröffneten Gefäss bei einem Patienten, einen in Relation zu weiteren Bereichen des Gefässes erhöhten Druck an eine Einstichstelle im Gefäss anzulegen. Mit anderen Worten, legt der Druckkörper 3 einen in Relation zur Einstichstelle im Gefäss reduzierten Druck an eine Punktionsstelle an der Haut und einen in Relation zur Einstichstelle im Gefäss reduzierten Druck an einem Bereich zur Pulsmessung am Gefäss an.

In dem in den Figuren gezeigten Beispiel ist der Druckkörper der anatomischen Form einer Fingernachbildung zum manuellen Verschliessen des eröffneten Gefässes nachgebildet. Genauer gesagt, entspricht die anatomische Form einer Nachbildung von drei Fingern. Hierbei steht die mittlere Fingernachbildung der insgesamt drei Fingernachbildungen in Relation zu den beidseitig anliegenden Fingernachbildungen anatomisch nachgebildet vor. Insgesamt ist die anatomische Form der Fingernachbildungen in Bezug auf eine Einstichstelle im Gefäss, in Bezug auf eine Punktionsstelle an der Haut und in Bezug auf einen Bereich der Pulsmessung an dem Gefäss abgestimmt.

Ferner sind zwei Haltebänder 4 als Ausläufer des Verschlusselements 2 an distalen Enden davon bereitgestellt. Die Haltebänder 4 haben unelastische Eigenschaften. Beispielsweise können die Haltebänder 4 einstückig mit dem Verschlusselement 2 ausgebildet sein. Um den Haltebändern 4 die unelastischen Eigenschaften zu vermitteln, ist beispielsweise eine Verstärkungsschicht 5 auf den Haltebändern 4 angebracht. Diese Verstärkungsschicht 5 ist wiederum aus einem Material mit unelastischen Eigenschaften ausgebildet. Die Verstärkungsschicht 5 kann mittels eines herkömmlichen (technischen) Klebemittels 6 auf die Haltebänder 4 aufgeklebt werden. In einem Fall, bei welchem die Haltebänder 4 aus Silikon hergestellt sind, kann es notwendig sein, die Kontaktfläche mit dem Klebemittel 6 in geeigneter Weise vorzubereiten. Um den dauerhaften Kontakt von Silikon mit dem verwendeten Klebemittel 6 zu verbessern, kann deshalb eine Oberflächenbehandlung, wie z.B. eine Plasma-, Corona-, nasschemische- oder sonstige Behandlung, vorgesehen sein.

Alternativ kann das gemeinsame Material, aus welchem das Verschlusselement 2, der Druckkörper 3 und die Haltebänder 4 einstückig ausgebildet sind, derart durch beispielsweise gezielte Aushärte-Verarbeitungsabläufe bearbeitet werden, dass lediglich nur das Material im Bereich der Haltebänder 4 keine Dehnung zulässt, während die restlichen Bereiche weiterhin dehnbar sind.

Obwohl in der Zeichnung nicht gezeigt, können die Haltebänder 4 und das Verschlusselement 2 aus unterschiedlichen Materialien bestehen, welche miteinander verbunden sind. In diesem Beispiel kann das Verschlusselement 2 aus Silikon bestehen und können die Haltebänder 4 aus Polyethylen (PE) bestehen.

An der Unterseite der Verstärkungsschicht 5 ist eine Hautkleberschicht 7 zum Aufkleben auf die Haut bereitgestellt. Die Hautkleberschicht 7 hat vorzugsweise hautkleberspezifische Eigenschaften. Schliesslich kann die Hautkleberschicht 7 zusätzlich noch mit einer abziehbaren Schutzfolie versehen sein (nicht dargestellt).

Ferner enthält das Gefässverschlusssystem 1 in dem Bereich des Verschlusselementes 2 zwei Indikatorelemente 8, welche mit dem Verschlusselement 2 verbunden sind. In einer Ausführungsform sind die Indikatorelemente 8 einstückig mit dem Verschlusselement 2 ausgebildet. Die Indikatorelemente 8 sind hierbei beidseitig vom Druckkörper 3, vorzugsweise bei gleichen Abständen, angeordnet. Die Indikatorelemente 8 sind dazu ausgelegt, eine mit zunehmender Streckung durch Anlegen einer Zugkraft auf das Gefässverschlusssystem 1 (und somit auf das Verschlusselement 2) veränderliche Geometrie anzunehmen, welche Veränderlichkeit in Relation zur Streckung und somit zur Zugkraft steht.

Das Verschlusselement 2, der wenigstens eine anatomisch nachgebildete Druckkörper 3, die Haltebänder 4, die Indikatorelemente 8 und die Verstärkungsschicht 5 sind vorzugsweise transparent oder annähernd transparent ausgebildet, um das Aufbringen des ortsdefiniert aufklebbaren Gefässverschlusssystems 1 zu erleichtern.

Wie bereits erwähnt, können das Verschlusselement 2, der wenigstens eine Druckkörper 3, die Haltebänder 4 und die Indikatorelemente 8 aus einem einstückigen Silikonmaterial bestehen. Neben der ausserordentlichen Dehnbarkeit, weist Silikon auch eine erwünschte hohe Rückstellkraft auf. Es können aber auch andere Materialien, wie z.B. Naturkautschuk, Kunstkautschuk, Gummi, Latex, Hydrogel, polymerer Kunststoff oder eine Kombination dieser Materialien, verwendet werden. Alternativ können die Haltebänder aus Polyethylen (PE) hergestellt sein.

Der anatomisch nachgebildete Druckkörper 3 hat im Normalfall (beispielsweise nach einer Untersuchung und/oder Behandlung, usw.) direkten Hautkontakt. Deshalb sind für die verwendeten Materialien geeignete medizinische Eigenschaften, vor allem hinsichtlich der Haut- und Körperverträglichkeit, erforderlich.

Anhand von Fig. 3 wird die Verwendung des ortsdefiniert aufklebbaren Gefässverschlusssystems 1 zum Verschliessen eines eröffneten Gefässes beschrieben.

Bevor eine Nadel oder Kanüle 9 aus der Einstichstelle entnommen wird, wird die entsprechende Körperstelle auf herkömmliche Weise gereinigt und desinfiziert. Anschliessend werden die Schutzfolien (in den Figuren nicht dargestellt) von beiden Haltebändern des Gefässverschlusssystems 1 abgezogen. Das Gefässverschlusssystem 1 wird dann auf die betreffende Körperstelle mittels der Haltebänder derart angelegt und fixiert, dass der Druckkörper 3 später bei der vollständigen Applikation des Gefässverschlusssystems 1, wie in Fig. 3 durch einen Pfeil angedeutet, derart zum Liegen kommt, dass ein maximaler Druck an der Einstichstelle des Gefässes angelegt wird. Zudem kommt der Druckkörper 3 derart zum Liegen, dass ein erhöhter Druck an der Punktionsstelle an der Haut des Patienten angelegt wird. Ferner wird ein erhöhter Druck an einen Bereich zur Pulsmessung am Gefäss angelegt. Hierbei ist zu beachten, dass die beiden zuletzt genannten Drücke geringer sind als der Druck, welcher an der Einstichstelle des Gefässes angelegt wird. Hierdurch wird, analog zum medizinisch fachgemässen manuellen Abdrücken eines eröffneten Gefässes, die Einstichstelle des Gefässes maximal abgedrückt, während, in Relation hierzu, die Punktionsstelle an der Haut reduziert abgedrückt wird, um somit die ungehinderte Blutzufuhr zu gewährleisten. Anschliessend wird die Nadel, Kanüle 9 Schleuse oder ein Katheter entnommen.

Als Einsatzgebiete für das Gefässverschlusssystem 1 seien genannt: Gefässverschlusssystem zum Verschliessen von Gefässen nach interventionellen Eingriffen mit Kathetern über die Femoralis, Cubitalis oder Brachialis; in der Allgemeinmedizin vor dem Entfernen von Verweilkanülen; in der Gefässchirurgie nach dem Entfernen von Varizen; nach minimalinvasiven, chirurgischen Eingriffen vor dem Entfernen von medizinischen Gerätschaften.

Bei der genannten Anwendung des erfindungsgemässen Gefässverschlusssystems als Verschlusssystem zum Verschliessen der Femoralis, wird das Gefässverschlusssystem z.B. im Anschluss an eine Koronarintervention angelegt. Koronarinterventionen werden fast ausschliesslich über die Femoralis durchgeführt. Arterielle Verschlusssysteme wurden mit dem Ziel entwickelt, die Hämostasezeit zu verkürzen und gleichzeitig periphere Gefässkomplikationen zu vermeiden bzw. zu reduzieren. Eine im Vergleich zum Stand der Technik weiter verkürzte Hämostasezeit, bei gleichzeitiger Verhinderung peripherer Gefässkomplikationen, ist durch das erfindungsgemässe Gefässverschlusssystem erzielt.

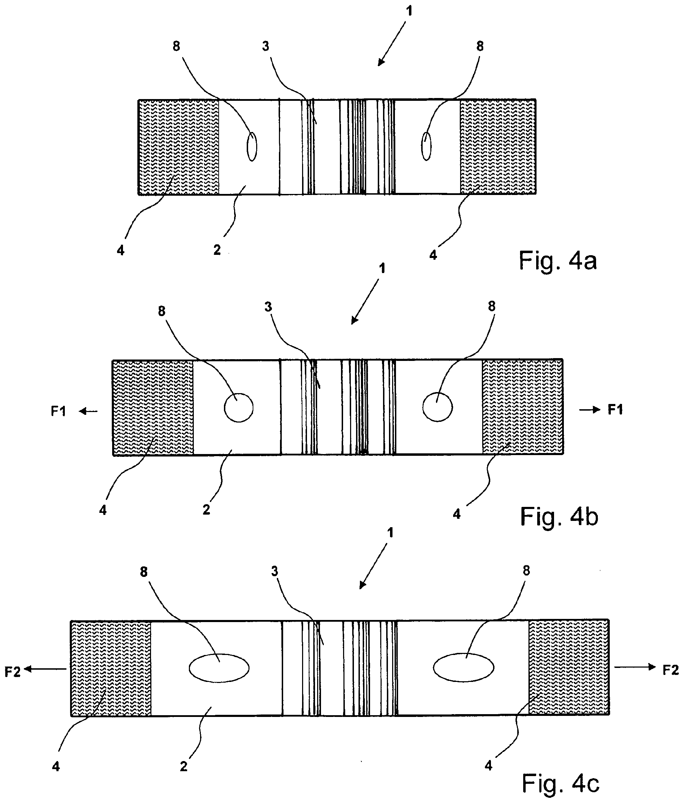

In Figuren 4a-c sind schematische Ansichten des Gefässverschlusssystems 1 bei unterschiedlichen Streckungen gezeigt. Die Indikatorelemente 8 sind in dieser Ausführungsform einstückig mit dem Verschlusselement 2 ausgebildet. Die Indikatorelemente 8 können beispielsweise in Relation zum Verschlusselement 2 als Aussparung oder Hervorhebung ausgeführt sein. In einem weiteren Beispiel können die Indikatorelemente 8 mittels Materialabtrag bzw. Materialauftrag an lediglich der Kontur der Indikatorelemente 8 ausgeführt werden. Es können auch mehr als zwei Indikatorelemente 8 vorgesehen sein.

Im ursprünglichen, unbelasteten Zustand des Gefässverschlusssystems 1 (siehe Fig. 4a) sind die Indikatorelemente 8 mit der Geometrie einer ovalen Form ausgebildet. Das Zentrum der Indikatorelemente 8 sollte jeweils in einem Abschnitt des Verschlusselements 2 in der Mitte zwischen dem anatomisch nachgebildeten Druckkörper 3 und dem Ansatz zum Halteband 4 zum Liegen kommen. Es sind auch andere Formen als die ovale Form für die Indikatorelemente 8 denkbar.

Durch ein Strecken des Gefässverschlusssystems 1 durch Anlegen von jeweils einer Zugkraft F1 an die Haltebänder 4, wird aufgrund der elastischen Eigenschaft des Materials des Verschlusselements 2 lediglich dieses Element in die Länge gestreckt. Durch diese Streckung werden auch die Indikatorelemente 8 gestreckt, mit dem Ergebnis, dass deren ovale Form zunehmend in eine kreisförmige Form übergeht, wie in Fig. 4b gezeigt.

Durch Erhöhen der Zugkräfte von F1 auf F2, geht die kreisförmige Form in eine ovale Form über, wie in Fig. 4c gezeigt. Durch Betrachten der jeweiligen Form der Indikatorelemente 8 ist der Benutzer dazu in der Lage, einen Rückschluss auf die jeweilige Zugkraft zu ziehen. In Ansprechen hierauf ist der Benutzer somit dazu in der Lage, den jeweiligen Druck definieren zu können, welchen der Druckkörper 3 eines derart gestreckten Gefässverschlusssystems 1 an das eröffnete Gefäss, an die Einstichstelle der Haut und an einen Bereich zur Pulsmessung am Gefäss anlegt, sobald das derart gestreckte Gefässverschlusssystem 1 auf die Haut des Patienten aufgeklebt ist. Somit kann der Druck schnell, einfach und anwendungsspezifisch definiert werden.

## Patentansprüche

1. Gefässverschlusssystem (1) mit einem Verschlusselement (2) und wenigstens einem Druckkörper (3), wobei das Verschlusselement (2) aus einem Material mit elastischen Eigenschaften ausgebildet ist; und mit
einer Vielzahl von Haltebändern (4), welche an distalen Enden des Verschlusselementes (2) angeschlossen sind, wobei die Haltebänder (4) unelastische Eigenschaften haben; und mit vorzugsweise zumindest einem Indikatorelement (8), welches mit dem Verschlusselement (2) verbunden ist; und wobei der wenigstens eine Druckkörper (3) ausgelegt ist, im angelegten Zustand an einem eröffneten Gefäss bei einem Patienten, einen in Relation zu weiteren Bereichen des Gefässes erhöhten Druck an eine Einstichstelle im Gefäss anzulegen, **dadurch gekennzeichnet, dass** der wenigstens eine Druckkörper (3) im rechten Winkel zur Längserstreckung der Haltebänder (4) angeordnet ist und dabei eine treppenförmige Aussenform hat, wobei eine grösste Erhebung der treppenförmigen Aussenform dazu dient, diese Einstichstelle im Gefäss abzudrücken, während weitere Erhebungen der treppenförmigen Aussenform dazu ausgelegt sind, weitere Bereiche am Gefäss abzudrücken.

2. Gefässverschlusssystem (1) nach Anspruch 1, **dadurch gekennzeichnet, dass** der wenigstens eine Druckkörper (3) ausgelegt ist, einen in Relation zur Einstichstelle im Gefäss reduzierten Druck an eine Punktionsstelle an der Haut anzulegen.

3. Gefässverschlusssystem (1) nach Anspruch 1 oder 2, **dadurch gekennzeichnet, dass** der wenigstens eine Druckkörper (3) ausgelegt ist, einen in Relation zur Einstichstelle im Gefäss reduzierten Druck an einen Bereich zur Pulsmessung am Gefäss anzulegen.

4. Gefässverschlusssystem (1) nach einem der vorhergehenden Ansprüche, **dadurch gekennzeichnet, dass** der Druckkörper der anatomischen Form einer Fingernachbildung zum manuellen Verschliessen des eröffneten Gefässes nachgebildet ist.

5. Gefässverschlusssystem (1) nach Anspruch 4, **dadurch gekennzeichnet, dass** die anatomische Form einer Nachbildung von drei Fingern entspricht.

6. Gefässverschlusssystem (1) nach Anspruch 5, **dadurch gekennzeichnet, dass** die mittlere Fingernachbildung der drei Fingernachbildungen in Relation zu den beidseitig anliegenden Fingernachbildungen anatomisch nachgebildet vorsteht.

7. Gefässverschlusssystem (1) nach Anspruch 5 oder 6, **dadurch gekennzeichnet, dass** die anatomische Form der Fingernachbildungen in Bezug auf eine Einstichstelle im Gefäss, eine Punktionsstelle an der Haut und einen Bereich der Pulsmessung an dem Gefäss abgestimmt ist.

8. Gefässverschlusssystem (1) nach einem der Ansprüche 1 bis 3, **dadurch gekennzeichnet, dass** die treppenförmige Aussenform des Druckkörpers (3) mit zwei weiteren Erhebungen ausgelegt ist, um diese weiteren Bereiche am Gefäss abzudrücken.

9. Gefässverschlusssystem (1) nach Anspruch 1, **dadurch gekennzeichnet, dass** das Verschlusselement (2) und die Haltebänder (4) einstückig oder mehrstückig ausgebildet sind.

10. Gefässverschlusssystem (1) nach einem der Ansprüche 1 bis 9, **dadurch gekennzeichnet, dass** das Verschlusselement (2) und/oder der wenigstens eine Druckkörper (3) und/oder das zumindest eine Indikatorelement (8) einstückig ausgebildet sind.

## Claims

1. A vessel closure system (1) comprising a closure element (2) and at least one pressure body (3), wherein the closure element (2) comprising a material with elastic properties; and with
a plurality of holding bands (4), which are joining on the distal ends of the closure element (2), wherein the holding bands (4) having unelastic properties; and
with preferable at least one indicator element (8), which is connected with the closure element (2); and
wherein the at least one pressure body (3) is construed in the laid in condition in an opened vessel of a patient to create to additional areas of the vessel an increased pressure at a puncture position in the vessel, **characterised in that** the at least one pressure body (3) is arranged at right angle to the longitudinal extension of the holding bands (4) and having a stair shaped outer form, wherein a largest protrusion of the stair shaped outer form is serving to close the puncture in the vessel, while additional protrusions of the stair shaped outer form are construed for closing further areas at the vessel.

2. The vessel closure system (1) according to Claim 1, **characterised in that** the at least one pressure body (3) is construed to create a reduced pressure in relation to the puncture in the vessel thus on a puncture position on the skin.

3. The vessel closure system (1) according to Claim 1 or 2, **characterised in that** the at least one pressure body (3) is construed to create in relation to the puncture a reduced pressure on an area for the measurement of the pulse frequency.

4. The vessel closure system (1) according to any of the preceding Claims, **characterised in that** the pressure body is made as a replica of the anatomical shape of finger replica for a manual closure of the opened vessel.

5. The vessel closure system (1) according to Claim 4, **characterised in that** the replica of an anatomical shape complies on three fingers.

6. The vessel closure system (1) according to Claim 5, **characterised in that** the middle finger replica of the three finger replica protrudes in relation to the fingers adjacent on either side anatomical replicated.

7. The vessel closure system (1) according to Claim 5 or 6, **characterised in that** the anatomical shape of the finger replicas is coordinated in relation to a puncture in the vessel, a puncture position on the skin and an area of the measurement of the pulse frequency at the vessel,

8. The vessel closure system (1) according to any of the preceding Claims 1 to 3, **characterised in that** the stair shaped outer form of the pressure body (3) is construed with two further protrusions to close the additional areas of the vessel.

9. The vessel closure system (1) according to Claim 1, **characterised in that** the closure element (2) and the holding bands (4) are made in one piece or in a number of pieces.

10. The vessel closure system (1) according to any of the preceding Claims 1 to 9, **characterised in that** the closure element (2) and/or the at least one pressure body (3) and/or the at least one indicator element (8) are made in one piece.

## Revendications

1. Système (1) de fermeture de vaisseau, comprenant un élément (2) de fermeture et au moins un corps (3) d'application d'une pression, l'élément (2) de fermeture étant en un matériau ayant des propriétés élastiques et comprenant une pluralité de rubans (4) de maintien, qui sont raccordés aux extrémités distales de l'élément (2) de fermeture, les rubans (4) de maintien ayant des propriétés inélastiques et comprenant de préférence au moins un élément (8) indicateur, qui est relié à l'élément (2) de fermeture, le au moins un corps (3) d'application d'une pression étant conçu pour, à l'état appliqué sur un vaisseau ouvert chez un patient, appliquer, en un point de ponction du vaisseau, une pression plus grande qu'en d'autres parties du vaisseau, **caractérisé en ce que** le au moins un corps (3) d'application d'une pression est disposé à angle droit par rapport à l'étendue longitudinale des rubans (4) de maintien et a ainsi une forme extérieure en escalier,
la surélévation la plus grande de la forme extérieure en escalier servant à presser ce point de ponction du vaisseau, tandis que d'autres surélévations de la forme extérieure en escalier sont conçues pour presser d'autres parties du vaisseau.

2. Système (1) de fermeture de vaisseau suivant la revendication 1, **caractérisé en ce que** le au moins un corps (3) d'application d'une pression est conçu pour appliquer à la peau, en un point de ponction, une pression réduite par rapport au point de ponction du vaisseau.

3. Système (1) de fermeture de vaisseau suivant la revendication 1 ou 2, **caractérisé en ce que** le au moins un corps (3) d'application d'une pression est conçu pour appliquer au vaisseau, à une partie de mesure du pouls, une pression réduite par rapport au point de ponction du vaisseau.

4. Système (1) de fermeture de vaisseau suivant l'une des revendications précédentes, **caractérisé en ce que** le corps d'application d'une pression est reproduit à la forme anatomique d'une reproduction de doigt pour la fermeture manuelle du vaisseau ouvert.

5. Système (1) de fermeture de vaisseau suivant la revendication 4, **caractérisé en ce que** la forme anatomique correspond à une reproduction de trois doigts.

6. Système (1) de fermeture de vaisseau suivant la revendication 5, **caractérisé en ce que** la reproduction de doigt médiane des trois reproductions de doigt est en saillie en reproduction de manière anatomique par rapport aux reproductions de doigt se trouvant des deux côtés.

7. Système (1) de fermeture de vaisseau suivant la revendication 5 ou 6, **caractérisé en ce que** la forme anatomique des reproductions de doigt est déterminée, par rapport au point de ponction dans le vaisseau, à un point de ponction sur la peau et à une région de mesure du pouls sur le vaisseau.

8. Système (1) de fermeture de vaisseau suivant l'une des revendications 1 à 3, **caractérisé en ce que** la forme extérieure en escalier du corps (3) d'application d'une pression est conçue en ayant deux autres surélévation pour appuyer ces autres régions sur le vaisseau.

9. Système (1) de fermeture de vaisseau suivant la revendication 1, **caractérisé en ce que** l'élément (2) de fermeture et les rubans (4) de maintien sont constitués en une pièce ou en plusieurs pièces.

10. Système (1) de fermeture de vaisseau suivant l'une des revendications 1 à 9, **caractérisé en ce que** l'élément (2) de fermeture et/ou le au moins un corps (3) d'application d'une pression et/ou le au moins un élément (B) indicateur sont constitués d'une seule pièce.
